# FASCICULE DE BREVET EUROPEEN

(11) **EP 0 807 420 B1**
(45) Date de publication et mention de la délivrance du brevet: **31.07.2002**
(21) Numéro de dépôt: 97401057.1
(22) Date de dépôt: 13.05.1997
(51) Int. Cl.: A61B 17/70

(54) **Dispositif de fixation osseuse, en particulier au sacrum, en ostéosynthèse du rachis**
Knochenbefestigungsvorrichtung, insbesondere des Kreuzbeines, für die Osteosynthese der Wirbelsäule
Bone fixation device, particularly of the sacrum, for spinal osteosynthesis

(30) Priorité: 13.05.1996 FR 9605898
(43) Date de publication de la demande: 19.11.1997
(73) Titulaire: Stryker France S.A., 93290 Tremblay-en-France (FR); Ganem, Franck, 14000 Caen (FR)
(72) Inventeur: Ganem, Franck, 14000 Caen (FR)
(74) Mandataire: Le Forestier, Eric

(56) Documents cités:
- EP-A- 0 201 024
- EP-A- 0 625 337
- EP-A- 0 654 249
- US-A- 5 443 467

## Description

La présente invention a trait d'une façon générale à un dispositif de fixation, en particulier au sacrum, utilisable en ostéosynthèse du rachis.

Il est courant de pratiquer des ostéosynthèses du rachis en utilisant des dispositifs de fixation prenant ancrage dans le sacrum. Il est ainsi possible de procéder, entre les différents étages fixés, à des mouvements de réduction qui peuvent être des contractions, des distractions ou encore des rappels de vertèbres ayant glissé vers l'avant du rachis. Cette dernière pathologie est connue sous le nom de "spondylolisthésis".

Dans le cas d'une réduction de spondylolisthésis, une distraction est souvent pratiquée. Une réduction partielle est ainsi obtenue par la remise en tension des fibres de l'appareil ligamentaire et discal du patient. La réduction est complétée par l'utilisation du matériel selon la technique opératoire qui lui est associée. Cette opération est réalisée, sauf cas exceptionnel, de manière bilatérale. Dans toute la description qui suit, on s'attachera à décrire un des côtés du traitement.

La plupart des plaques ou blocs de fixation au sacrum connus dans l'art antérieur présentent certains inconvénients. L'un de ces inconvénients réside en ce que l'orientation des vis d'ancrage osseux est imposée, ce qui donne au produit un manque de souplesse d'utilisation évident compte-tenu des variations de configuration du sacrum et de son voisinage d'un patient à l'autre.

Un autre inconvénient des plaques ou blocs de fixation au sacrum connus réside dans le risque d'un dévissage des vis d'ancrage sous l'effet notamment des micro-mouvements relatifs se produisant entre les matériels et le sacrum. Il en résulte alors la faillite de la fixation sacrée.

Par ailleurs, les ostéosynthèses du rachis sont réalisées grâce à des matériels permettant de fixer, de facon souvent très rigide, différentes unités fonctionnelles de la colonne vertébrale. Il en découle une variation brutale de la répartition des efforts, et donc des états de contraintes et de déformations résultants, au niveau des disques sus- et sous-jacents à la fixation. Cette modification entraîne à plus ou moins long terme des dégénérescences discales. Cette pathologie est connue sous le nom de "syndrome de charnière".

On connaît par ailleurs du document EP-A-0 625 337 un dispositif selon le préambule de la revendication 1, qui permet de faire varier l'inclinaison des vis d'ancrage par rapport à un élément de liaison allongé, de même que l'espacement entre ces vis.

Ce dispostif connu est toutefois désavantageux en ce que les moyens de liaison allongés sont constitués de plusieurs composants, à savoir un organe allongé en forme de cuvette et une pluralité de blocs de fixation répartis le long de l'organe allongé et recevant chacun une tête de vis et, en partie inférieure, la zone correspondante de l'organe allongé.

Il en résulte que la manipulation et la pose de ce dispositif par le chirurgien sont extrêmement fastidieuses, avec en particulier la nécessité de faire glisser sur l'organe allongé une série de blocs de fixation, et de les y maintenir en position.

La présente invention vise donc à pallier cet inconvénient.

Elle propose à cet effet un dispositif de fixation osseuse tel que défini dans la revendication 1.

Des aspects préférés, mais non limitatifs, du dispositif selon l'invention sont définis dans les revendications dépendantes.

D'autres aspects, buts et avantages de la présente invention apparaîtront mieux à la lecture de la description détaillée suivante de formes de réalisation préférées de celle-ci, donnée à titre d'exemple et faite en référence aux dessins annexés, sur lesquels :
la figure 1 est une vue en coupe longitudinale médiane d'un élément de fixation selon l'invention,
la figure 2 est une vue de face de l'élément de la figure 1,
la figure 3 est une vue de côté de l'élément ainsi que de trois vis d'ancrage osseux et de bouchons associés,
la figure 4 est une vue de face de l'élément pourvu des vis et des bouchons,
la figure 5 est une vue en élévation de côté d'un bouchon destiné à coopérer avec l'élément des figures 1 et 2 et avec une vis d'ancrage osseux,,
la figure 6 est une vue en élévation de côté de la vis d'ancrage osseux,
la figure 7 est une vue en coupe transversale de l'élément de fixation, d'une vis d'ancrage osseux et du bouchon associé, après montage,
la figure 8 est une vue partiellement en élévation et partiellement en coupe d'une vis d'ancrage osseux auxiliaire destinée à coopérer avec une autre partie de l'élément de fixation des figures 1 et 2,
les figures 9 à 12 sont des vues en coupe transversale analogues à la figure 7, de quatre variantes de réalisation de l'invention,
la figure 13 est une vue en coupe transversale des différents composants d'une cinquième variante de réalisation de l'invention, et
la figure 14 est une vue en coupe transversale de cette cinquième variante à l'état assemblé.

On notera à titre préliminaire que, d'une figure à l'autre, des éléments ou parties identiques ou similaires sont désignés dans la mesure du possible par les mêmes signes de référence, et ne seront pas décrits à chaque fois.

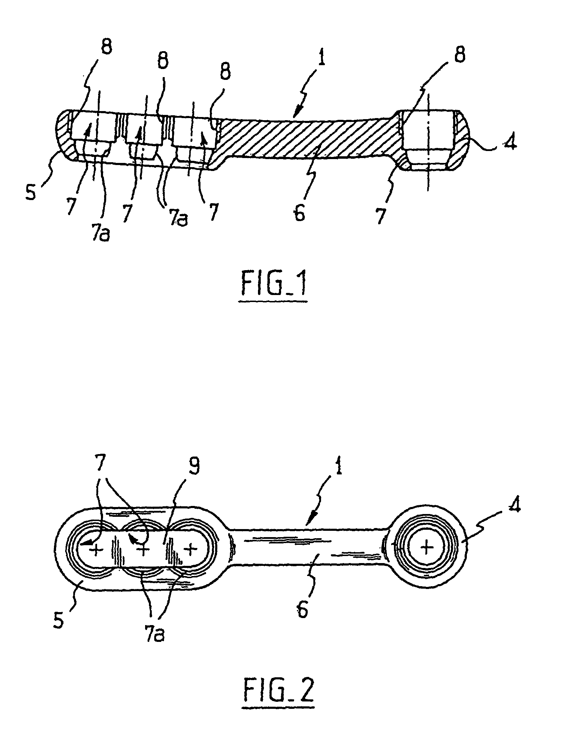

En référence maintenant aux figures 1 et 2, on a représenté un élément de fixation au sacrum, globalement désigné par la référence 1, qui se compose de trois parties, à savoir un oeillet supérieur 4, une plaque sacrée 5 et une tige 6, par exemple cylindrique, reliant l'oeillet à la plaque. L'élément 1 présente dans son ensemble, en profil, une courbure en lordose illustrée sur la figure 1.

Dans la plaque 5 est formée une série de trous 7 alignés dans le sens de la longueur de l'élément 1, pour des vis d'ancrage osseux 2.

De préférence, on peut proposer au chirurgien plusieurs modèles d'éléments 1, avec notamment différentes longueurs de tige 6, et dans la plaque 5, différents nombres de trous pour vis d'ancrage osseux.

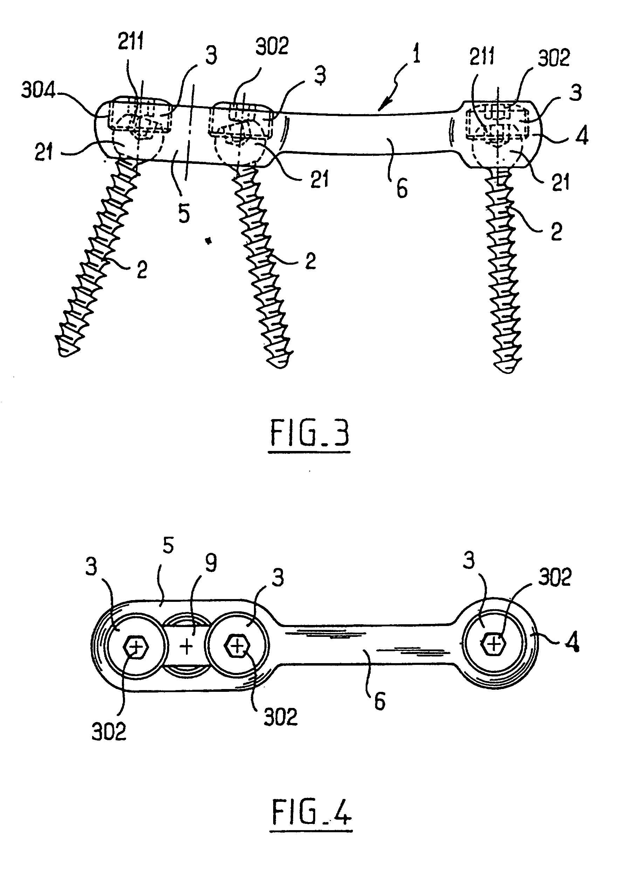

Comme le montre la figure 3, la plaque 5 peut recevoir, dans une version à trois trous 7, deux vis 2 d'ancrage osseux et, dans une version à cinq trous (non illustrée), trois vis 2 d'ancrage osseux.

Les trous 7 7 communiquent entre eux par le biais d'une rainure traversante 9 de section oblongue, comme on peut le voir sur la figure 2. Cette rainure permet de faire passer aisément une vis 2 d'une position à une autre au cours de l'opération de réduction. Chacun de ces trous comporte, dans la région de son fond, une cupule sphérique 7a destinée à coopérer avec une partie inférieure complémentaire de la tête 21 de la vis 2, cette tête 21 étant dans son ensemble sphérique et comportant une empreinte de serrage 211 par exemple du type à six pans hexagonaux.

Cette conformation de chaque trou 7 et de la tête de vis associée 21 permet un débattement angulaire de la vis dans un angle solide dont le demi-angle au sommet est par exemple de l'ordre de 30°.

Par ailleurs, chaque vis étant stabilisée dans son logement au cours de la pose, deux vis distantes sont aptes à maintenir une distraction osseuse sans nécessiter l'utilisation d'un instrument distracteur.

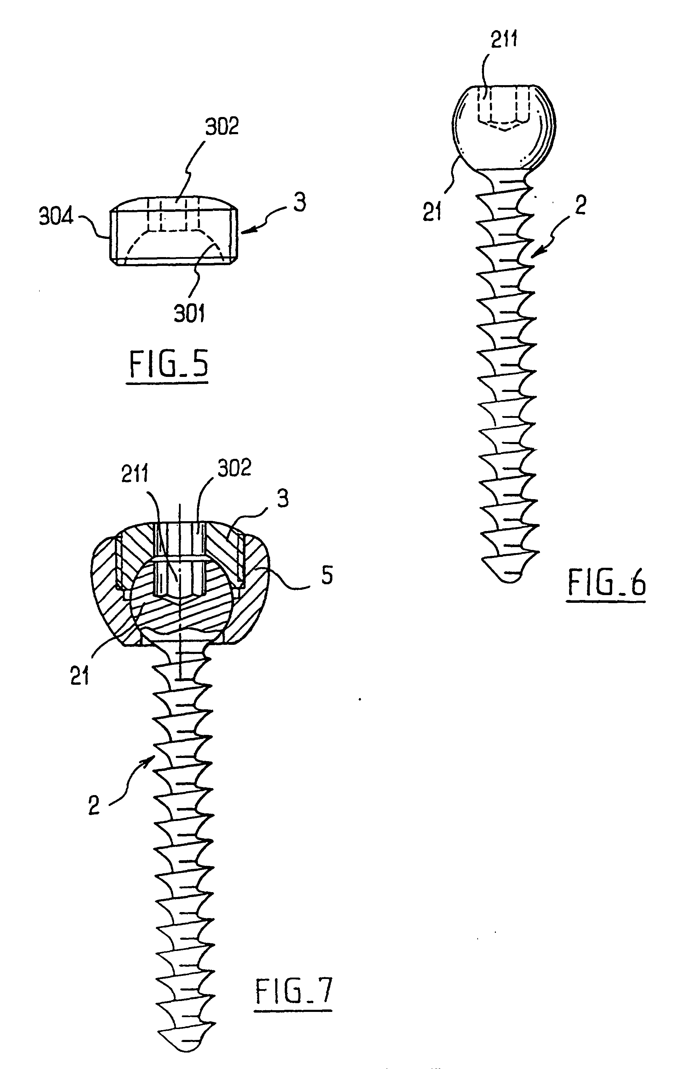

Comme le montrent en particulier les figures 1 et 7, chaque trou 7 possède intérieurement, à distance de la cupule 7a, un taraudage 8 lui permettant de recevoir un bouchon 3 représenté en détail sur la figure 5. Ce bouchon comporte sur sa face extérieure une empreinte de serrage 302, de préférence identique à celle de la tête de vis 21 de manière à pouvoir utiliser le même outil de serrage, et un logement en cupule sphérique 301 coopérant avec le sommet également sphérique de la tête de vis 21. Le bouchon comporte également à sa périphérie un filetage 304 destiné à coopérer avec le filetage 8 de l'orifice 7. Ce bouchon 3 a pour objet, après vissage et serrage, d'assurer le blocage de la vis 2 dans la position angulaire déterminée par le chirurgien.

La plaque 5, la vis 2 et le bouchon 3 après assemblage sont illustrés notamment sur la figure 7. Le bouchon fileté 3 assure une fonction anti-retour empêchant tout dévissage accidentel de la vis d'ancrage 2 associée, notamment dans les conditions exposés en préambule de la présente demande.

En outre, grâce au dispositif de la présente invention, le spondylolisthésis peut être réduit en implantant dans le pédicule de la vertèbre concernée soit une vis intermédiaire fixée sur la tige de liaison intermédiaire 6, soit une vis placée dans l'oeillet 4.

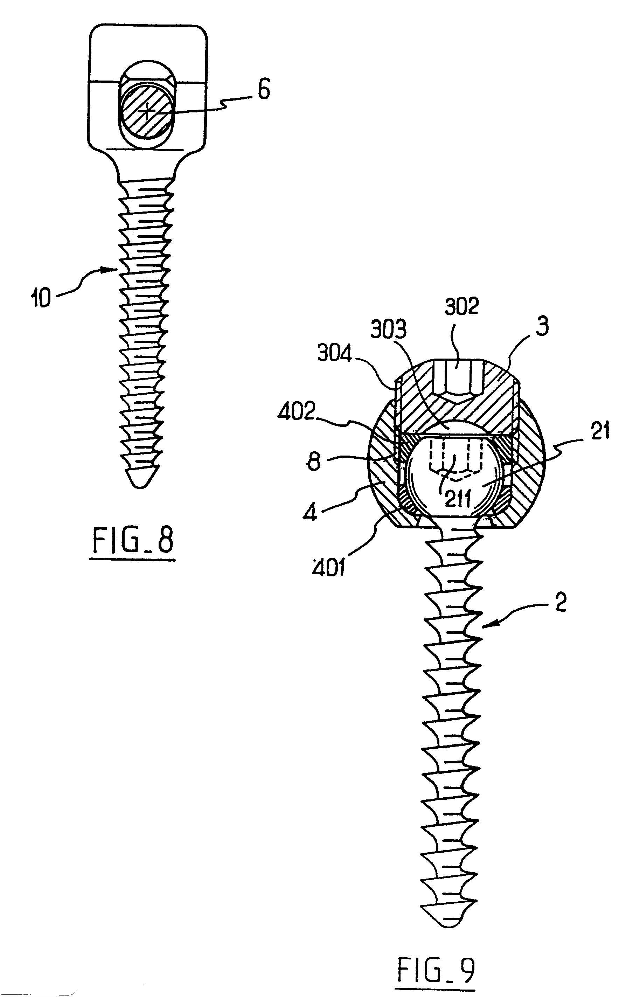

Dans le premier cas, la tige intermédiaire 6 peut ainsi être utilisée pour effectuer un rappel de vertèbre. A cet effet, et comme le montre la figure 8, on peut y associer une vis à chargement supérieur, globalement désignée par la référence 10 et connue en soi, qui aura été préalablement implantée dans la vertèbre en question.

On peut également solidariser la vertèbre à solliciter à la tige intermédiaire 6 à l'aide d'un autre type de dispositif, non illustré mais également connu en soi, dans lequel la vis est décalée par rapport à l'axe de la tige 6 mais reliée à cette dernière par un svstème de maintien.

Dans le second cas, c'est l'oeillet supérieur 4 de l'élément 1 qui est utilisé pour recevoir une vis d'ancrage osseux 2 dans la vertèbre à traiter, cette vis 2 étant de préférence de même type que celle décrite ci-dessus. A cet effet, un trou 7 identique à celui décrit en référence à la plaque 5 est formé dans ledit oeillet, pour permettre là encore une angulation de la vis 2 et une fonction anti-retour permettant grâce à un bouchon 3 d'éviter qu'elle ne se desserre.

Afin de ne pas entraîner le syndrome de charnière décrit en préambule, on peut éviter d'assurer un strict blocage de la vis 2 introduite dans l'oeillet supérieur 4. On conserve alors dans ce cas une mobilité relative de la vis 2 par rapport à l'oeillet 4.

De cette manière, la position de la vertèbre dans laquelle est ancrée la vis 2 introduite dans l'oeillet 4 est fixée, mais la fonctionnalité du disque intervertébral sous-jacent est en partie maintenue. On crée ainsi un gradient de rigidité dans la fixation qui a pour effet d'atténuer le brusque changement d'état de contraintes au niveau du disque sus-jacent.

Cette mobilité relative de la vis 2 dans l'oeillet 4 peut être assurée de différentes manières.

Une première solution, non illustrée, consiste à reprendre le principe illustré sur la figure 7 en utilisant un bouchon fileté 3 et en prévoyant dans le trou 7 de l'oeillet un épaulement empêchant le bouchon 3 d'atteindre une position de blocage de la vis 2 en position angulaire.

En variante, également non illustrée, un tel épaulement peut être formé sur le bouchon fileté 3.

En outre, ou alternativement, on peut diminuer jusqu'à rendre négligeable le frottement entre le bouchon 3 et la tête 21 de la vis 2 en appliquant en couche mince un revêtement à faible coefficient de friction, par exemple de type céramique ou carbone diamant, sur une ou plusieurs des différentes surfaces en contact, à savoir la tête de vis 21, la cupule 7a du trou 7 de l'oeillet 4, et la cupule 301 du bouchon fileté 3.

Par exemple, on peut appliquer un tel revêtement seulement sur la tête 21 de la vis 2.

Maintenant en référence aux figures 9 à 14, on va décrire un certain nombre de variantes de réalisation permettant d'atténuer la rigidité de l'assemblage de la vis 2 et de l'oeillet 4 dans lequel elle est introduite, afin là encore d'éviter le syndrome de charnière.

Ainsi la figure 9 décrit une solution dans laquelle tout contact entre les pièces rigides devant adopter des mouvements relatifs est évité par l'utilisation d'une première bague torique 401, inférieure, et d'une seconde bague torique 402, supérieure. Ces deux bagues sont de préférence réalisées en un matériau amortissant tel que du silicone, et sont illustrées sur la figure 9 à l'état déformé après serrage.

Ainsi, lorsque les bagues 401, 402 sont mises en pression et déformées lors du serrage, elles assurent une liaison à rigidité atténuée entre la vis 2 et l'oeillet 4.

Avantageusement, on met à disposition du chirurgien des bagues possédant différentes nuances de dureté, tout en conservant les mêmes dimensions, ce qui lui permet de moduler la rigidité de l'assemblage sans avoir à jouer sur le couple de serrage du bouchon fileté 3, ce qui s'avèrerait très délicat à mettre en oeuvre de façon fiable.

En outre, pour minimiser les frottements entre les bagues 401, 402 et la tête 21 de la vis, il peut être avantageux de réaliser sur la vis un traitement de surface par bombardement ionique à l'azote, qui permet d'améliorer les caractéristiques tribologiques de la tête de vis.

Toujours en référence à la figure 9, on observe que le bouchon 3 possède ici encore une cupule sphérique 303, destinée seulement à permettre l'angulation de la vis 2, mais sans appui bloquant sur la tête 21 de ladite vis.

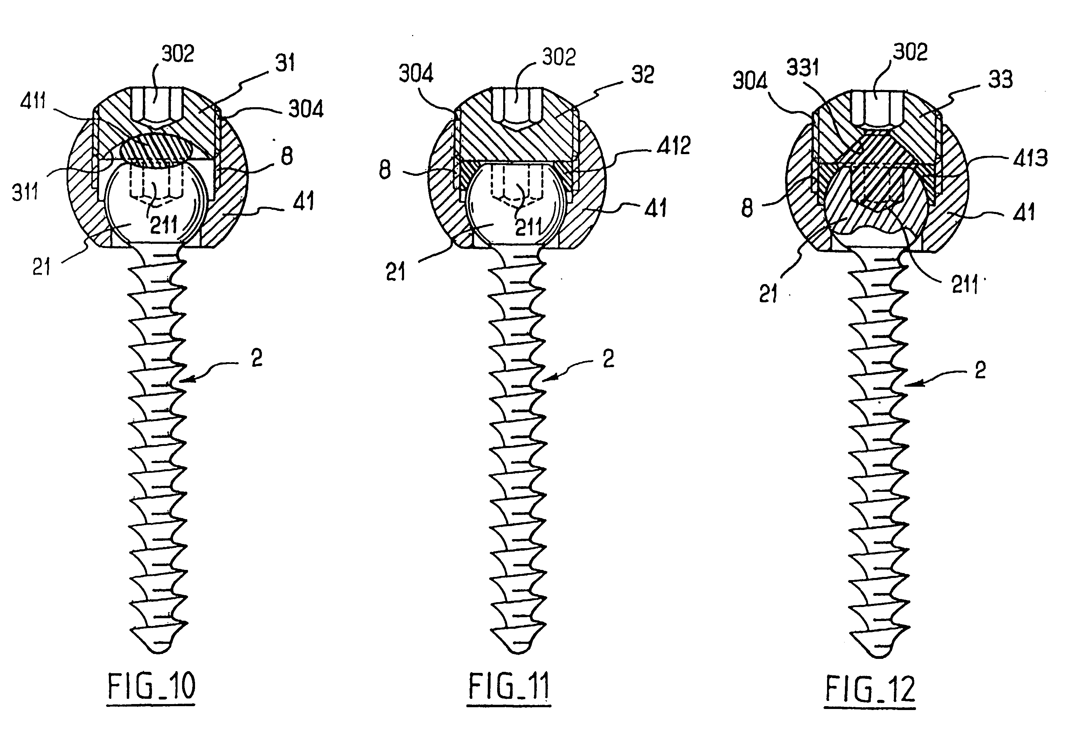

La figure 10 décrit une variante dans laquelle le bouchon 31 se distingue de celui de la figure 5 en ce que sa cupule, indiquée en 311, n'est plus complémentaire de la tête sphérique 21 de la vis 2, mais est évasée, tandis qu'une bille 411 en matériau amortissant est interposée entre la cupule 311 et ladite tête 21, et mise en pression et déformée lors du serrage.

On assure ici encore une atténuation de la rigidité d'assemblage.

La figure 11 décrit une variante dans laquelle le bouchon fileté 32 présente une surface de travail plane, et dans laquelle le contact direct entre le bouchon 32 et la tête de vis 21 est supprimé par l'utilisation d'une bague torique 412 réalisée dans un matériau amortissant, mise en pression et déformée lors du serrage.

La figure 12 décrit une variante dans laquelle le bouchon, désigné par la référence 33, présente sur sa face de travail un renfoncement conique 331, et dans laquelle le contact entre le bouchon 33 et la tête de vis 21 est supprimé par l'injection, au cours de l'opération de pose, d'un fluide tyxotropique 413 assurant une fonction d'amortissement.

En outre, dans les trois variantes de réalisation des figures 10, 11 et 12 le frottement de contact entre la tête sphérique 21 et la cupule 7a du trou 7 formé dans l'oeillet 4 peut également être réduit par l'utilisation d'un revêtement tel que cité plus haut. A cet égard, le revêtement utilisé dans la variante de la figure 12 sera choisi de manière à ce que le contact entre la base de la tête sphérique 21 et la cupule 7a assure l'étanchéité du fluide tyxotropique mis sous pression lors du vissage.

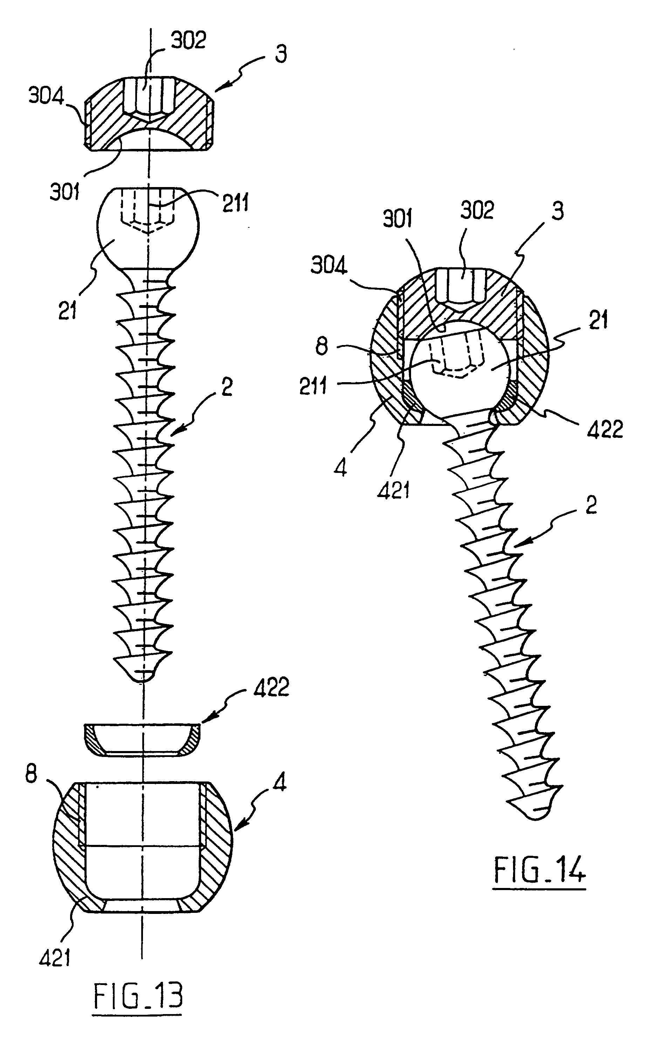

Les figures 13 et 14 décrivent une autre variante de réalisation dans laquelle l'oeillet est modifié. Plus précisément, l'oeillet 4 présente une cuvette généralement cylindrique 421 dont la partie éloignée du fond est filetée en 8 pour recevoir le bouchon fileté 3, identique à celui de la figure 5.

Entre la cuvette 421 et la tête de vis 21 est interposé un anneau 422. Il peut s'agir soit d'un anneau rigide, réalisé par exemple en alliage de titane revêtu d'une céramique, ou en alliage de titane traité par bombardement ionique, ou encore de céramique massive, soit encore d'une bague torique identique à la bague 401 utilisée dans la variante de réalisation de la figure 9, réalisée là encore en un matériau amortissant, par exemple du silicone:

Dans le cas où l'anneau 422 est rigide, il peut être avantageux de donner au rayon de courbure de sa portée sphérique intérieure une valeur légèrement inférieure au rayon de la tête de vis 21, de manière à assurer une mise en contrainte de ladite tête de vis lors du serrage. Dans ce cas, la version des figures 13 et 14 peut avantageusement être utilisée également lorsqu'un blocage total de la vis est souhaité.

Selon une autre variante, l'anneau 422 peut être fendu, avec là encore une mise en contrainte de la tête 21 et une possibilité de blocage.

Naturellement, les pièces tels que l'élément 1, les vis 2, les bouchons 3 sont réalisées en tout matériau biocompatible de propriétés mécaniques adaptées, tel qu'un alliage de titane.

On donnera ci-dessous pour terminer quelques observations complémentaires.

Tout d'abord la forme et les dimensions de l'élément 1 sont choisies de façon à permettre de résister aux contraintes de torsion grâce à l'appui plan engendré entre la base de la partie 5 en forme de plaque et l'os adjacent.

Cette forme permet également de limiter les mouvements de translation longitudinqale sur les vis intermédiaires.

Ensuite, les moyens d'atténuation de la rigidté décrits plus haut assurent une telle atténuation à la fois dans le plan sagittal et dans le plan transversal.

Enfin l'invention permet de proposer un système d'ostéosynthèse présentant une grande modularité : ainsi, par exemple, différentes versions de l'anneau 422 peuvent être proposées au praticien pour gérer la zone de contact antérieure (côté vertèbre), tandis que l'un des moyens d'amortissement décrits plus haut peut être proposé avec différents degrés de rigidité pour gérer l'amortissement en partie postérieure (côté bouchon). Dans la pratique, on peut ainsi couvrir toute la gamme entre les liaisons souples et les liaisons rigides.

A cet égard, on observera que les moyens d'atténuation de rigidité décrits plus haut peuvent être utilisés aussi bien au niveau de la vis de l'oeillet 4 qu'au niveau des vis de la plaque 5.

## Revendications

1. Dispositif de fixation osseuse, notamment au sacrum pour ostéosynthèse du rachis, comprenant des moyens de liaison allongés recevant au moins deux vis (2), d'ancrage dans un os, au moins une première des vis traversant un orifice (7) formé dans les moyens de liaison, dans lequel les moyens de liaison comportent dans la région de leur fond une portée de section transversale essentiellement circulaire (7a), dans lequel la tête (21) de la première vis comporte une surface essentiellement sphérique d'appui contre ladite portée, et dans lequel les moyens de liaison comportent au niveau de l'orifice un premier filetage (8), le dispositif comprenant en outre un bouchon (3) comportant un second filetage (304) apte à coopérer avec le premier filetage et à venir en contact de serrage avec ladite tête de vis (21) pour la maintenir dans une position angulaire souhaitée, dispositif **caractérisé en ce que** les moyens de liaison sont constitués par un élément en forme de plaque d'un seul tenant (5) dans laquelle sont ménagés l'orifice (7) et le premier filetage (8), et **en ce que** ladite portée (7a) est essentiellement sphérique.

2. Dispositif selon la revendication 1, **caractérisé en ce que** le premier filetage est un taraudage (8) formé intérieurement dans l'orifice à distance dudit fond, et **en ce que** le bouchon (3) est fileté extérieurement (304).

3. Dispositif selon l'une des revendications 1 et 2, **caractérisé en ce que** la tête de vis (21) présente en outre un sommet généralement sphérique, et **en ce que** le bouchon (3) comporte intérieurement une cavité sphérique (301) complémentaire.

4. Dispositif selon la revendication 1 ou 2, **caractérisé en ce que** ladite plaque (5) comporte une série d'orifices (7) alignés et reliés entre eux par une rainure (9) permettant le déplacement d'une vis d'ancrage osseux (2) d'un orifice à l'autre avant serrage.

5. Dispositif selon l'une des revendications 1 à 4, **caractérisé en ce que** ladite tête de vis (21) et ledit bouchon (3) comportent des empreintes de serrage identiques (211, 302).

6. Dispositif selon l'une des revendications 1 à 5, **caractérisé en ce que** ladite plaque (5) est destinée à une fixation dans un premier os et fait partie d'un élément d'un seul tenant (1) comportant en outre un oeillet (4) pour une autre vis (2) d'ancrage osseux dans un second os, et une tige (6) reliant ladite plaque audit oeillet.

7. Dispositif selon la revendication 6, **caractérisé en ce qu'**il comprend en outre un dispositif (10) d'ancrage osseux dans un autre os, sur lequel est fixée ladite tige (6).

8. Dispositif selon l'une des revendications 1 à 7, **caractérisé en ce qu'**il comprend au moins deux vis (2) d'ancrage osseux traversant des orifices respectifs (7) formés dans ladite plaque (5), et **en ce que** les deux vis sont aptes à prendre deux positions angulaires différentes.

9. Dispositif selon l'une des revendications 1 à 8, **caractérisé en ce qu'**il comprend en outre des moyens (401, 402; 411; 412; 413; 422) pour atténuer la rigidité de l'assemblage entre au moins l'une des vis (2) d'ancrage et ledit élément (1).

10. Dispositif selon la revendication 9, **caractérisé en ce que** lesdits moyens d'atténuation de la rigidité comprennent au moins une pièce en matériau souple (402; 411; 412) interposée et comprimée entre ladite tête de vis (21) et le bouchon associé (3).

11. Dispositif selon la revendication 10, **caractérisé en ce qu'**y est associée une pluralité de pièces en matériau souple de souplesses différentes, de manière à permettre différents degrés d'atténuation de la rigidité sans avoir à jouer sur le couple de serrage du bouchon (3).

12. Dispositif selon la revendication 10 ou 11, **caractérisé en ce que** ladite pièce est une bague torique (402, 412).

13. Dispositif selon la revendication 10 ou 11, **caractérisé en ce que** ladite pièce est une bille torique (411).

14. Dispositif selon la revendication 9, **caractérisé en ce que** lesdits moyens d'atténuation de la rigidité comprennent un fluide tyxotropique (413) interposé et mis sous pression entre le bouchon (3) et ladite tête de vis (21).

15. Dispositif selon l'une des revendications 1 à 14, **caractérisé en ce que** ladite portée sphérique dudit orifice est formée par déformation d'une bague torique (401) placée dans une région de fond dudit orifice (7).

16. Dispositif selon l'une des revendications 1 à 14, **caractérisé en ce que** ladite portée sphérique dudit orifice est constituée par une surface d'une bague rigide (422) rapportée dans une région de fond dudit orifice (7).

17. Dispositif selon la revendication 16, **caractérisé en ce que** ladite bague rigide (422) est réalisée en un matériau choisi dans le groupe comprenant les alliages de titane revêtus de céramique, les alliages de titane traités par bombardement ionique, et les céramiques massives.

18. Dispositif selon l'une des revendications 9 à 14, **caractérisé en ce que** les moyens d'atténuation de la rigidité comprennent en outre un revêtement de surface apte à diminuer les frottements entre ladite portée essentiellement sphérique (7a) et ladite surface complémentaire de la tête de vis (21).

19. Dispositif selon la revendication 18, **caractérisé en ce que** ledit revêtement de surface est réalisé par bombardement ionique à l'azote.

## Patentansprüche

1. Kaochenbefestigungsvorrichtung, insbesondere an das Kreuzbein, für die Osteosynthese der Wirbelsäule, welche längliche Verbindungsstücke umfasst, welche mindestens zwei Verankerungsschrauben (2) in einem Knochen aufnehmen, wobei mindestens eine erste der Schrauben eine Öffnung (7) durchquert, welche sich in den Verbindungsstücken befindet, und in welcher die Verbindungsstücke in ihrem Bodenbereich eine Passung von im wesentlichen kreisförmigen Querschnitt (7a) tragen, und in welcher der Kopf (21) der ersten Schraube eine im wesentlichen kugelformige Oberfläche als Stütze gegen die besagte Passung hat, und in welcher die Verbindungsstücke auf Höhe der Öffnung ein erstes Gewinde haben (8), wobei die Vorrichtung darüber hinaus einen Verschluss (3) umfasst, welcher ein zweites Gewinde (304) hat, das geeignet ist, mit dem ersten Gewinde verschraubt und mit dem besagten Schraubenkopf (21) verspannt zu werden, um diesen in einer gewünschten Winkelposition zu halten, Vorrichtung, **dadurch gekennzeichnet, dass** die Verbindungsstücke aus einem Element in Form einer Platte aus einem Stück (5) bestehen, in welcher die Öffnung (7) und das erste Gewinde (8) untergebracht sind, und dass die besagte Passung (7a) im wesentlichen kugelförmig ist.

2. Vörrichtung nach Anspruch 1, **dadurch gekennzeichnet, dass** das erste Gewinde ein Gewinde (8) im Innern der Öffnung mit Abstand zum besagten Boden ist und dass der Verschluss (3) außen ein Gewinde eingeschnitten hat (304).

3. Vorrichtung nach einem der Ansprüche 1 und 2, **dadurch gekennzeichnet, dass** der Schraubenkopf (21) darüber hinaus eine im allgemeinen kugelförmige Spitze darstellt und dass der Verschluss (3) im Innern einen komplementären kugelförmigen Hohlraum (301) hat.

4. Vorrichtung nach Anspruch 1 oder 2, **dadurch gekennzeichnet, dass** die besagte Platte (5) eine Reihe von aneinandergereihten Öffnungen (7) trägt, welche untereinander durch eine Nut (9) verbunden sind, welche die Umsetzung einer Knochenverankerungsschraube (2) von einer in eine andere Öffnung vor dem Verschrauben erlaubt.

5. Vorrichtung nach einem der Ansprüche 1 bis 4, **dadurch gekennzeichnet, dass** der besagte Schraubenkopf (21) und der besagte Verschluss (3) identische Einsenkungen zum Verschrauben haben (211, 302).

6. Vorrichtung nach einem der Ansprüche 1 bis 5, **dadurch gekennzeichnet, dass** die besagte Platte (5) dazu bestimmt ist, in einem ersten Knochen befestigt zu werden, und dass sie ein Teil eines Elementes aus einem Stück (1) darstellt, welches des weiteren eine Öse (4) für eine andere Schraube (2) zur Knochenverankerung in einem zweiten Knochen trägt und einen Schaft (6), welcher die besagte Platte mit der besagten Öse verbindet.

7. Vorrichtung nach Anspruch 6, **dadurch gekennzeichnet, dass** sie des weiteren eine Vorrichtung (10) zur Knochenverankerung in einem anderen Knochen umfasst, auf welcher der besagte Schaft befestigt wird (6).

8. Vorrichtung nach einem der Ansprüche 1 bis 7, **dadurch gekennzeichnet, dass** sie zumindest zwei Knochenverankerungsschrauben (2) umfasst, welche jeweilige Öffnungen (7) durchqueren, welche sich in der besagten Platte (5) befinden, und dass die beiden Schrauben in der Lage sind, zwei verschiedene Winkelpositionen einzunehmen.

9. Vorrichtung nach einem der Ansprüche 1 bis 8, **dadurch gekennzeichnet, dass** sie des weiteren Mittel (401, 402, 411, 412, 413, 422) enthalten, um die Starrheit der Verbindung zwischen mindestens einer der Verankerungsschrauben (2) und dem besagten Element (1) zu abzuschwächen.

10. Vorrichtung nach Anspruch 9, **dadurch gekennzeichnet, dass** die besagten Mittel zum Abschwächen der Starrheit mindestens ein Teil aus einem flexiblen Material (402, 411, 412) umfassen, welches zwischen dem besagten Schraubenkopf (21) und dem zugehörigen Verschluss (3) eingefügt und komprimiert wird.

11. Vorrichtung nach Anspruch 10, **dadurch gekennzeichnet, dass** eine Vielzahl von Teilen aus flexiblem Material verschiedener Flexibilität hinzugefügt ist, so dass verschiedene Grade der Abschwächung der Starrheit ermöglicht werden, ohne dass man das Anzugsmoment des Verschlusses (3) verändern muß.

12. Vorrichtung nach Anspruch 10 oder 11, **dadurch gekennzeichnet, dass** das besagte Teil eine torische Buchse darstellt (402, 412).

13. Vorrichtung nach Anspruch 10 oder 11, **dadurch gekennzeichnet, dass** das besagte Teil einen torischen Rundkörper darstellt (411).

14. Vorrichtung nach Anspruch 9, **dadurch gekennzeichnet, dass** die besagten Mittel zum Abschwächen der Starrheit einen tyxotropen flüssigen Körper (413) umfassen, welcher zwischen dem Verschluss (3) und dem besagten Schraubenkopf (21) eingefügt und unter Druck gesetzt wird.

15. Vorrichtung nach einem der Ansprüche 1 bis 14, **dadurch gekennzeichnet, dass** die besagte kugelförmige Passung der besagten Öffnung durch die Deformation einer torischen Buchse (401) gebildet wird, welche im unteren Bereich der besagten Öffnung (7) eingesetzt wird.

16. Vorrichtung nach einem der Ansprüche 1 bis 14, **dadurch gekennzeichnet, dass** die besagte kugelförmige Passung der besagten Öffnung durch die Oberfläche einer starren Buchse (422) gebildet wird, welche im unteren Bereich der besagten Öffnung (7) wieder eingefügt wird.

17. Vorrichtung nach Anspruch 16, **dadurch gekennzeichnet, dass** die besagte starre Buchse (422) aus einem Material hergestellt wird, welches aus der Gruppe der mit Keramik überzogenen Titanlegierungen, der mit Ionenbeschuss behandelten Titanlegierungen und der massiven Keramiken ausgewählt wurde.

18. Vorrichtung nach einem der Ansprüche 9 bis 14, **dadurch gekennzeichnet, dass** die Mittel zum Abschwächen der Starrheit unter anderem eine Oberflächenbeschichtung haben, welche geeignet ist, die Reibungen zwischen der besagten im wesentlichen kugelförmigen Passung (7a) und der besagten komplementären Oberfläche des Schraubenkopfes (21) zu verringern.

19. Vorrichtung nach Anspruch 18, **dadurch gekennzeichnet, dass** die besagte Oberflächenbeschichtung durch Ionenbeschuss in Stick stoff hergestellt wird.

## Claims

1. A bone fixing device, in particular for fixing to the sacrum for osteosynthesis of the backbone, the device comprising elongate link means receiving at least two bone-fastening screws (2), at least a first one of the screws passing through an orifice (7) formed in the link means, in which the link means include in the bottom region thereof a bearing surface (7a) of essentially circular cross-section, in which the head (21) of the first screw has a surface that is essentially spherical for bearing against said bearing surface, and in which the link means include a first thread (8) in the orifice, the device also including a plug (3) having a second thread (304) suitable for co-operating with the first thread and for coming into clamping contact with said screw head (21) to hold it in a desired angular position, the device being **characterized in that** the link means are constituted by a single-piece plate-shaped element (5) having the orifice (7) and the first thread (8) formed therein, and **in that** said bearing surface (7a) is essentially spherical.

2. A device according to claim 1, **characterized in that** the first thread is a tapping (8) formed internally in the orifice at a distance from said bottom, and the plug (3) has an outside thread (304).

3. A device according to claim 1 or 2, **characterized in that** the screw head (21) also has a generally spherical top, and the plug (3) has a complementary internal spherical cavity (301).

4. A device according to claim 1 or 2, **characterized in that** said plate (5) includes a series of orifices (7) in alignment that are interconnected by a slot (9) enabling a bone-fastening screw (2) to be moved from one orifice to another before tightening.

5. A device according to any one of claims 1 to 4, **characterized in that** said screw head (21) and said plug (3) have identical tightening sockets (211, 302).

6. A device according to any one of claims 1 to 5, **characterized in that** said plate (5) is designed to be fixed to a first bone and forms an integral portion of an element (1) that also includes an eyelet (4) for another bone-fastening screw (2) for fastening to a second bone, together with a rod (6) interconnecting said plate and said eyelet.

7. A device according to claim 6, **characterized in that** it further includes a bone-fastening device (10) for fastening to another bone, to which said rod (6) is fixed.

8. A device according to any one of claims 1 to 7, **characterized in that** it includes at least two bone-fastening screws (2) passing through respective orifices (7) formed in said plate (5), and the two screws are suitable for taking up two different angular positions.

9. A device according to any one of claims 1 to 8, **characterized in that** it further includes means (401, 402; 411; 412; 413; 422) for attenuating the stiffness of the assembly between at least one of the bone-fastening screws (2) and said element (1).

10. A device according to claim 9, **characterized in that** said means for attenuating stiffness comprise at least one piece of flexible material (402; 411; 412) interposed and compressed between said screw head (21) and the associated plug (3).

11. A device according to claim 10, **characterized in that** it is associated with a plurality of pieces of flexible material having different flexibilities, thereby enabling different degrees of stiffness attenuation to be provided without requiring action to be taken on the torque with which the plug (3) is tightened.

12. A device according to claim 10 or 11, **characterized in that** said piece is an O-ring (402, 412).

13. A device according to claim 10 or 11, **characterized in that** said piece is a pellet (411).

14. A device according to claim 9, **characterized in that** said stiffness attenuation means comprise a thixotropic fluid (413) interposed between and compressed between the plug (3) and said screw head (21).

15. A device according to any one of claims 1 to 14, **characterized in that** said spherical bearing surface of said orifice is formed by deforming an O-ring (401) placed in a bottom region of said orifice (7).

16. A device according to any one of claims 1 to 14, **characterized in that** said spherical bearing surface of said orifice is constituted by a surface of a rigid ring (422) fitted in a bottom region of said orifice (7).

17. A device according to claim 16, **characterized in that** said rigid ring (422) is made of a material selected from the group comprising: ceramic-coated titanium alloys, titanium alloys treated by ion bombardment, and solid ceramics.

18. A device according to any one of claims 9 to 14, **characterized in that** the stiffness attenuation means further include a surface coating suitable for reducing friction between said essentially spherical bearing surface (7a) and said complementary surface of the screw head (21).

19. A device according to claim 18, **characterized in that** said surface coating is made by nitrogen ion bombardment.
